# EUROPÄISCHE PATENTSCHRIFT

(11) **EP 0 950 394 B1**
(45) Veröffentlichungstag und Bekanntmachung des Hinweises auf die Patenterteilung: **21.01.2004**
(21) Anmeldenummer: 99101407.7
(22) Anmeldetag: 26.01.1999
(51) Int. Cl.: A61K 7/13

(54) **Verfahren und Mittel zum oxidativen Färben von Haaren**
Method and means for the oxidative dyeing of hair
Procédé et moyens pour la teinture d'oxydation des cheveux

(30) Priorität: 31.03.1998 DE 29805893 U
(43) Veröffentlichungstag der Anmeldung: 20.10.1999
(73) Patentinhaber: Spring Kosmetik GmbH & Co. KG, 33628 Bielefeld (DE)
(72) Erfinder: Schrader, Karheinz Dr., 37603 Holzminden (DE)
(74) Vertreter: Schmitz, Hans-Werner, Dipl.-Ing.

(56) Entgegenhaltungen:
- EP-A- 0 542 129
- EP-A- 0 755 669
- EP-B- 0 624 362
- WO-A-87/01033
- WO-A-88/01162
- WO-A-94/04125
- US-A- 5 112 360

## Beschreibung

Die Erfindung betrifft ein saures Haarfärbemittel mit einem neuartigen Färbungskatalysator sowie ein Verfahren zum oxidativen Färben von Haaren, bei dem unmittelbar vor der Anwendung aus einer Entwicklerlotion und einer Farbmasse ein Haarfärbemittel zubereitet, auf das zu färbende Haar aufgetragen und zur Einwirkung gebracht wird, worauf das Haarfärbemittel nur noch aufemulgiert und ausgespült werden muß.

Für die dauerhafte Färbung von Haar werden alkalische Oxidationsfarbstoffe verwendet, die unmittelbar vor der Anwendung mit einem sauren Oxidationsmittel, z.B. Wasserstoffperoxid, zum gebrauchsfertigen, auf das zu färbende Haar aufzutragenden Haarfärbepräparat vermischt werden. Die Färbung entsteht hierbei durch Oxidation bestimmter Entwicklersubstanzen mit bestimmten Kupplersubstanzen in Gegenwart eines geeigneten Oxidationsmittels. Das gebrauchsfertige Präparat liegt dabei deutlich im alkalischen Bereich, wodurch die Oberflächen der zu färbenden Haare in einer für das Eindringen der Oxidationsfarbstoffe vorteilhaften Weise aufgeschlossen werden. Aber die alkalischen Präparate beanspruchen die Haare auch, so daß es leicht bei wiederholter Anwendung zu Schädigungen des Haares kommt.

Versuche, die schädigende Wirkung der alkalischen Haarfärbemittel durch saure Einstellung des applikationsfertigen Präparats abzumildern oder ganz zu verhindern gehen bislang immer auf Kosten der Farbqualität und Dauerhaftigkeit der Färbung.

Saure Haarfärbemittel sind aus den EP 0770 375 A1, EP 0663 204 A1, und aus der EP 0542 129 B1 bekannt. Die genannten Druckschriften betreffen saure Haarfärbemittel mit neuen Entwickler-Kupplersubstanzen. Die Qualität der damit erreichbaren Färbungen kann jedoch noch verbessert werden.

In der EP 0 624 362 B1 wird ein neuartiges Verfahren zur Färbung von Haaren vorgestellt, bei dem mit einem basischen Färbemittel kurz, um die Haare nicht zu sehr zu strapazieren, vorgefärbt wird und dann, nach einer vorhergegangenen Haarwäsche, mit einem sauren Haarfärbemittel nachgefärbt wird. Nachteilig an diesem Verfahren ist, daß zwischen den beiden Färbeschritten das Haar shampooniert werden muß.

Es ist ein saures Haarfärbemittel aus der WO 89/06531 bekannt, das mit Hilfe von Mikrokapseln immer nur geringe Mengen an Säure während des Färbevorgangs freisetzt, so daß die Färbung durch die pH-Wert-Erniedrigung möglichst wenig beeinträchtigt wird.

Aus den WO 87/01033, WO 88/01161 und Wo 88/1162 schließlich sind saure Haarfärbemittel bekannt, die zur Verbesserung ihrer Färbequalität Katalysatoren einsetzen. Dabei werden Mangandioxid, Salze ein- und zweiwertiger Metalle der ersten und zweiten Hauptgruppe und letztlich überhaupt Metallsalze als Katalysatoren vorgeschlagen. Nachteilig an diesen Färbemitteln ist jedoch, daß die Katalysatoren bei ihrer Anwendung extra zugemischt werden müssen und nicht lagerstabil in der Farbmasse und/oder in der Entwicklerlotion enthalten sind.

Es besteht demnach weiterhin ein Bedarf an sauren Haarfärbemitteln, die in Farbqualität und Dauerhaftigkeit der Färbung an die gewohnten alkalischen Haarfärbemittel heranreichen.

Aufgabe der Erfindung ist es daher, ein saures Haarfärbemittel und ein Verfahren zur Färbung von Haaren zu schaffen, bei dem das Haar mit wenig Aufwand dauerhaft und in guter Qualität schonend gefärbt wird.

Diese Aufgabe wird erfindungsgemäß dadurch gelöst, daß dem sauren Haarfärbemittel eine geringe Menge Metall als Katalysator für das Farbstoff-Kupplersystem zugesetzt ist, so daß eine Farbqualität und Dauerhaftigkeit der Färbung erzielt wird, die mit der von herkömmlichen, alkalischen Haarfärbemitteln vergleichbar ist und daß bei dem Verfahren zur Färbung der Haare einfaches Ausspülen des Haarfärbemittels genügt(ohne shampoonieren).

Gegenstand der Erfindung ist ein Mittel für die oxidative Färbung von Haaren, das unmittelbar vor der Anwendung durch Vermischen einer Entwicklerlotion mit einer Farbmasse hergestellt ist, wobei das applikationsfertige Mittel auf einen sauren pH-Wert eingestellt ist und als Katalysator für die Färbung eine geringe Menge Metall in feinverteilter Form enthält. Weiterhin ist Gegenstand der Erfindung ein Verfahren zur oxidativen Färbung von Haaren, bei dem unmittelbar vor der Anwendung aus einer Entwicklerlotion und einer Farbmasse ein saures, einen metallischen Katalysator enthaltendes Haarfärbemittel zubereitet, auf das zu färbende Haar aufgetragen, zur Einwirkung gebracht und in einem letzten Schritt das Haarfärbemittel mit Wasser aufemulgiert und ausgespült wird.

Bei einer vorteilhaften.Ausgestaltung der Erfindung umfaßt der metallische Katalysator ein Edelmetall oder eine Edelmetallegierung, wie z.B. Silber, Gold, Platin, Kupfer, Messing und/oder eine andere Legierung. Voraussetzung für die Anwendung des Metalls oder der Legierung als Katalysator ist selbstverständlich seine dermatologische Unbedenklichkeit, wie sie bei den Metallen, die traditionell zur Schmuckherstellung verwendet werden, gegeben ist.

Der metallische Katalysator ist vorteilhafterweise in einer Menge von 0,0001 bis 1 Gew%, bevorzugt 0,001 - 0,5 Gew% und besonders bevorzugt 0,005 - 0,1 Gew%, bezogen auf die Komponente (entweder Entwicklerlotion oder Farbmasse) in die er, bevorzugt lagerstabil, eingearbeitet ist, enthalten. Der Katalysator liegt, um optimale Wirksamkeit zu entfalten, feinverteilt mit möglichst großer spezifischer Oberfläche vor.

Der Katalysator ist vorteilhafterweise in der Farbmasse lagerstabil eingearbeitet, d.h. der Katalysator wird nicht unmittelbar vor Gebrauch zugegeben, sondern ist in der Farbmasse, wenn sie mit der Entwicklerlotion vermischt wird, bereits enthalten.

Als Metalle eignen sich alle dermatologisch unbedenklichen Metalle, wie z.B. Eisen, Mangan, Zink, Titan, Nickel, Kupfer, Aluminium, etc. und besonders die Schmuckmetalle Silber, Gold, Platin, Kupfer und Legierungen wie Messing etc.

Der pH-Wert des applikationsfertigen Haarfärbemittels liegt bei < 7, bevorzugt zwischen 5 und 7, besonders bevorzugt zwischen 6,5 und 7. Die Erfindung betrifft darüber hinaus ein Verfahren zur oxidativen Färbung von Haaren, bei dem unmittelbar vor der Anwendung aus einer Entwicklerlotion und einer Farbmasse ein saures, einen metallischen Katalysator enthaltendes Haarfärbemittel zubereitet, auf das zu färbende Haar aufgetragen, zur Einwirkung gebracht und in einem letzten Schritt das Haarfärbemittel aufemulgiert und ausgespült wird.

Bei der Durchführung des Verfahrens ist es vorteilhaft, wenn beim Vermischen der beiden Komponenten (gemeint sind Entwicklerlotion und Farbmasse) die Entwicklerlotion vorgelegt wird.

Bei einer bevorzugten Ausführungsform des Verfahrens wird das Haarfärbemittel durch Vermischen der Entwicklerlotion mit der Farbmasse im Verhältnis von 3:0,5 bevorzugt von 2,5:0,75 und besonders bevorzugt von 2:1 hergestellt.

Das erfindungsgemäße Verfahren wird vorteilhafterweise mit einer Einwirkzeit von 5 bis 25 Min, bevorzugt von 10 bis 20 Min durchgeführt, wobei zusätzlich Wärmequellen zur intensiveren Färbung verwendet werden können.

Als "Entwicklerlotion" wird vorliegend die, das Oxidationsmittel enthaltende, Komponente des zweikomponentigen Haarfärbemittels bezeichnet. Die Entwicklerlotion enthält neben dem Oxidationsmittel noch schwache Säuren, Stabilisatoren für das Wasserstoffperoxid, Emulsionsgrundlagen sowie übliche Zusatzstoffe und Additive.

Als "Farbmasse" wird vorliegend die, den Farbstoff enthaltende, Komponente bezeichnet, die aus einer üblichen Grundmasse und der farbgebenden Komponente (mit Farbstoff, Entwickler und Nuanceur) sowie üblichen Zusätzen und Additiven, besteht, wobei nach einer bevorzugten Ausführungsform der metallische Katalysator in die Grundmasse eingearbeitet ist.

Unter "Ausspülen" bei dem Verfahren wird verstanden, daß das Haarfärbemittel auf dem Kopf nach dem Färben mit Wasser aufemulgiert und dann sorgfältig ausgespült wird. Die Zugabe von Reinigungsmitteln entfällt.

Im folgenden wird die Erfindung anhand von beispielhaften Rezepturen näher erläutert (die angegebenen Namen richten sich nach der INCI-Nomenklatur):

### Beispiel für die Grundmasse:

| Name | Gew% |
|---|---|
| Cetearyl Alcohol | 16,0 |
| Ammonium Chloride | 7,2 |
| MIPA-Laurethsulfate, Cocamidopropyl Betain | 7,0 |
| Ceteareth-25 | 5,0 |
| PEG-2Hydrogenated Tallow Amine | 3,5 |
| EDTA | 1,0 |
| Butylene Glycol | 1,0 |
| Ceteareth-6, Stearyl Alcohol | 1,0 |
| Aqua dem. | 54,9 |
| Additive | 4,4 |
| Silber (silver) | 0,072 |

### Beispiel für die Entwicklerlotion:

| Name | Gew% |
|---|---|
| Aqua dem. | 89,1 |
| Hydrogen Peroxide | 6,7 |
| Cetylalkohol | 1,75 |
| Glyceryl Stearate, PEG-100 Stearate | 1,75 |
| sowie Additive und Stabilisatoren | ad 100 |

### Beispiele für Farbmassen:

### Beispiel 1: Haselnußbraun

| | |
|---|---|
| Pentylene Glykol | 2,0 |
| Toluene-2,5-Diamine Sulfate | 0,72 |
| Ethanolamine | 0,5 |
| 4-Amino-m-Cresol | 0,35 |
| Sodium Hydrosulfite | 0,073 |
| Sodium Picramate | 0,013 |
| 2-Amino-3-Hydroxypyridine | 0,01 |
| Aqua dem. | 26, 3 |
| Grundmasse | 70,0 |

<Perfluralkylethanol>
(der metallische Katalysator Silber ist in der Grundmasse mit 0,072 Gew% enthalten und damit in der Farbmasse mit 0,049 Gew%).

### Beispiel 2: Wildpflaume

| | |
|---|---|
| Pentylene Glykol | 2,0 |
| Toluene-2,5-Diamine Sulfate | 0,68 |
| Ethanolamine | 0,4 |
| 6-Amino-o-Cresol | 0,35 |
| Sodium Hydrosulfite | 0,07 |
| N,N'Bis (2-Hydroxyethyl)-2-Nitro-p-phenylenediamine | 0,60 |
| Fluowet EA 93 | 0,05 |
| Aqua dem. | 25,85 |
| Grundmasse | 70,00 |

### Beispiel 3: Polarsilberhell

| | |
|---|---|
| Pentylene Glykol | 1,0 |
| Toluene-2,5-Diamine Sulfate | 0,015 |
| Ethanolamine | 0,02 |
| Sodium Hydrosulfite | 0,003 |
| N,N'Bis(2-Hydroxyethyl)-2-Nitro-p-phenylenediamine | 0,025 |
| 1,2,4-Trihydroxybenzene | 0,01 |
| 1,5 Naphtalenediol | 0,0085 |
| Aqua dem. | 28,92 |
| Grundmasse | 70,00 |

<Fluowet EA 93>

## Patentansprüche

1. Mittel für die oxidative Färbung von Haaren, das unmittelbar vor der Anwendung durch Vermischen einer Entwicklerlotion mit einer Farbmasse hergestellt ist, wobei das applikationsfertige Mittel auf einen sauren pH-Wert eingestellt ist und einen Katalysator für die Färbung umfasst, wobei der Katalysator eine geringe Menge Metall in feinverteilter Form ist.

2. Mittel nach Anspruch 1, bei dem das Metall ein Edelmetall oder eine, ein Edelmetall enthaltende, Legierung ist.

3. Mittel nach Anspruch 1 oder 2, bei dem das Metall in einer katalytischen Menge von 0,0001 bis 1 Gew% enthalten ist.

4. Mittel nach einem der vorstehenden Ansprüche, bei dem das Metall in der Farbmasse lagerstabil eingearbeitet ist.

5. Verfahren zur oxidativen Färbung von Haaren, bei dem unmittelbar vor der Anwendung aus einer Entwicklerlotion und einer Farbmasse ein saures, einen metallischen Katalysator enthaltendes Haarfärbemittel zubereitet, auf das zu färbende Haar aufgetragen, zur Einwirkung gebracht und in einem letzten Schritt das Haarfärbemittel aufemulgiert und ausgespült wird.

6. Verfahren nach Anspruch 5, **dadurch gekennzeichnet, daß** beim Vermischen der beiden Komponenten (Entwicklerlotion und Farbmasse) die Entwicklerlotion vorgelegt wird.

7. Verfahren nach Anspruch 5 oder 6, **dadurch gekennzeichnet, daß** das Haarfärbemittel durch Vermischen der Entwicklerlotion mit der Farbmasse im Verhältnis von 3 : 0,5, vorzugsweise von 2,5 : 0,75 und besonders bevorzugt von 2 : 1 hergestellt wird.

8. Verfahren nach einem der Ansprüche 5 bis 7, **dadurch gekennzeichnet, daß** die Einwirkzeit 5 bis 25 Minuten, vorzugsweise 10 bis 20 Minuten beträgt, wobei vorzugsweise zusätzliche Wärmequellen zur intensiveren Färbung verwendet werden.

## Claims

1. Means for the oxidative dyeing of hair, which directly before application is prepared by mixing a developer lotion with a dyeing material, the application-ready means being set to an acid pH value and comprising a catalyst for dyeing, the catalyst being a small amount of metal in finely divided form.

2. The means according to claim 1, wherein the metal is a precious metal or an alloy containing a precious metal.

3. The means according to claim 1 or 2, wherein the metal is contained in a catalytic amount of 0.0001 to 1 % by wt.

4. The means according to any one of the preceding claims, wherein the metal is incorporated into the dyeing material in a storage-stable manner.

5. A method for the oxidative dyeing of hair, wherein directly before application an acid hair-dyeing means containing a metallic catalyst is prepared from a developer lotion and a color material and applied to the hair to be dyed and allowed to react and is emulsified and rinsed in a last step.

6. The method according to claim 5, **characterized in that** during mixing of the two components (developer lotion and color material) the developer lotion is supplied.

7. The method according to claim 5 or 6, **characterized in that** the hair dyeing means is prepared by mixing the developer lotion with the color material in the ratio of 3 : 0.5, preferably 2.5 : 0.75, and particularly preferably 2 : 1.

8. The method according to any one of claims 5 to 7, **characterized in that** the reaction time is 5 to 25 minutes, preferably 10 to 25 minutes, additional heat sources being preferably used for an enhanced dyeing.

## Revendications

1. Produit pour la teinture par oxydation de cheveux, lequel est préparé immédiatement avant l'application par mélange d'une lotion de développement avec une masse de teinture, le produit fini destiné à l'application étant réglé à une valeur de pH acide et contient un catalyseur pour la teinture, ledit catalyseur étant une faible quantité de métal sous forme finement divisée.

2. Produit selon la revendication 1, dans lequel le métal est un métal précieux ou un alliage contenant un métal précieux.

3. Produit selon l'une ou l'autre des revendications 1 et 2, dans lequel le métal est contenu dans une quantité catalytique de 0,0001 à 1 % en poids.

4. Produit selon l'une des revendications précédentes, dans lequel le métal est intégré dans la masse de teinture de manière stable vis-à-vis du stockage.

5. Procédé pour la teinture par oxydation de cheveux, dans lequel on prépare immédiatement avant l'application à partir d'une lotion de développement et d'une masse de teinture un produit de teinture acide contenant un catalyseur métallique, celui-ci est appliqué sur les cheveux à teindre, amené à agir, et dans une dernière opération, le produit de teinture est mis en émulsion et rincé.

6. Procédé selon la revendication 5, **caractérisé en ce que** lors du mélange des deux composants (lotion de développement et masse de teinture), la lotion de développement est mise en premier.

7. Procédé selon l'une ou l'autre des revendications 5 et 6, **caractérisé en ce que** le produit de teinture est préparé par mélange de la lotion de développement avec la masse de teinture dans un rapport de 3:0,5, de préférence de 2,5:0,75, et de manière particulièrement préférée de 2:1.

8. Procédé selon l'une des revendications 5 à 7, **caractérisé en ce que** le temps d'action s'élève de 5 à 25 minutes, de préférence de 10 à 20 minutes, et dans lequel on utilise de préférence des sources de chauffage additionnelles pour une teinture plus intensive.
